# EUROPEAN PATENT SPECIFICATION

(11) **EP 1 002 866 B1**
(45) Date of publication and mention of the grant of the patent: **12.10.2005**
(21) Application number: 98929834.4
(22) Date of filing: 03.07.1998
(51) Int. Cl.: C12N 15/31, C12N 1/21, C12P 13/08, C12P 13/14, C12N 9/00

(54) **TEMPERATURE SENSITIVE dtsR GENES**
TEMPERATUR-EMPFINDLICHE dtsR GENE
GENES DTSR SENSIBLES A LA TEMPERATURE

(30) Priority: 09.07.1997 JP 18417697
(43) Date of publication of application: 24.05.2000
(73) Proprietor: Ajinomoto Co., Inc., Tokyo 104-0031 (JP)
(72) Inventor: KIMURA, Eiichiro, Ajinomoto Co., Inc., Kawasaki-shi, Kanagawa 210-0801 (JP); YAGOSHI, Chizu, Ajinomoto Co., Inc., Kawasaki-shi, Kanagawa 210-0801 (JP); NAKAMURA, Jun, Ajinomoto Co., Inc., Kawasaki-shi, Kanagawa 210-0801 (JP); OSUMI, Tsuyoshi, Ajinomoto Co., Inc., Kawasaki-shi, Kanagawa 210-0801 (JP); NAKAMATSU, Tsuyoshi, Ajinomoto Co., Inc., Kawasaki-shi, Kanagawa 210-0801 (JP)
(74) Representative: HOFFMANN EITLE
(86) International application number: PCT/JP1998/003017
(87) International publication number: WO 1999/002692

(56) References cited:
- EP-A- 0 054 311
- EP-A- 0 780 477
- WO-A-95/23224
- WO-A-96/06180
- JP-A- 10 000 087
- KIMURA E ET AL: "A dtsR gene-disrupted mutant of Brevibacterium lactofermentum requires fatty acids for growth and efficiently produces L-glutamate in the presence of an excess of biotin." BIOCHEMICAL AND BIOPHYSICAL RESEARCH COMMUNICATIONS. UNITED STATES 8 MAY 1997, vol. 234, no. 1, 8 May 1997 (1997-05-08), pages 157-161, XP002219155 ISSN: 0006-291X
- KIMURA E. et al., "Molecular Cloning of a Novel Gene, dtsR, which Rescues the Detergent Sensitivity of a Mutant Derived from Brevibacterium Lactofermentum", BIOSCI. BIOTECH. BIOCHEM., Vol. 60, No. 10, (1996), pp. 1565-1570, XP002916460.

## Description

### Technical Field

The present invention relates to a temperature-sensitive dtsR gene, and specifically a gene encoding a protein which contributes to a surfactant resistance of coryneform bacteria and has a temperature-sensitive mutation. Also, the present invention relates to a coryneform bacterium having the gene and having an ability to produce L-lysine and L-glutamic acid, and a method for producing L-lysine and L-glutamic acid by fermentatative method using the coryneform bacterium.

### Background Art

L-Lysine and L-glutamic acid have been hitherto industrially produced by fermentatative methods by using coryneform bacteria belonging to the genus *Brevibacterium* or *Corynebacterium* having abilities to produce these amino acids. In these methods, it is known that the coryneform bacteria require biotin for their growth, while L-glutamic acid is not accumulated if an excessive amount of biotin exists in a medium. Therefore, any one of the following methods has been adopted in the conventional method of producing L-glutamic acid. Namely, cultivation is conducted in a medium in which the concentration of biotin is restricted, or cultivation is conducted such that a surfactant or a lactam antibiotic as a biotin action-suppressing agent is allowed to be contained in a medium at an initial or intermediate stage of cultivation in the case of use of the medium containing a sufficient amount of biotin. However, especially when a material such as waste molasses, which is inexpensive but contains an excessive amount of biotin, is used as a carbon source in a medium, the biotin action-suppressing agent, which is required to be added to the medium, has been a cause to increase the production cost.

In this regards, the present inventors have revealed the presence of a gene (*dts*R gene) which originates from coryneform bacteria and which encodes a protein (DTSR protein) imparting a surfactant resistance to the coryneform bacteria, and have found that L-glutamic acid-producing coryneform bacteria having the gene destroyed produce a considerable amount of L-glutamic acid under the condition that biotin is present at a concentration at which a wild type strain produces little L-glutamic acid and that L-glutamic acid-producing coryneform bacteria having L-lysine-producing ability is enhanced in the L-lysine producing ability when the *dts*R gene is amplified (International Publication No. WO95/23224).

Also, the present inventors have found that fermentative production of L-glutamic acid can be conducted stably even in the presence of biotin by imparting a temperature sensitivity to a biotin action-suppressing agent to L-glutamic acid-producing coryneform bacteria, and that simultaneous fermentative production of both of L-lysine and L-glutamic acid can be conducted stably even in the presence of biotin by imparting a L-glutamic acid-producing ability to the strain temperature-sensitive to the biotin action-suppressing agent. As a means of imparting the temperature sensitivity to the biotin action-suppressing agent to coryneform bacteria, gene substitution using a mutant type *dts*R gene encoding a temperature-sensitive mutant type DTSR protein is disclosed (International Publication No. WO96/06180).

### Disclosure of the Invention

As described above, methods for effectively using the *dts*R gene in the fermentative amino acid production using coryneform bacteria have been developed. The object of the present invention is to provide a novel mutant type *dts*R gene encoding a temperature-sensitive mutant type DTSR protein for a more effective use of the *dts*R gene.

The present inventors have succeeded in obtaining a novel mutant type *dts*R gene encoding a temperature-sensitive mutant type DTSR protein by subjecting an already obtained *dts*R gene to a mutation treatment and have accomplished the present invention.

Thus the present invention provides a DNA encoding:
(A) a protein which has an amino acid sequence shown in SEQ ID NO: 2, or an amino acid sequence shown in SEQ ID NO: 2 in which a leucine residue at position 139 is changed to an amino acid residue other than a proline residue, said protein having a temperature-sensitive DTSR activity, or
(B) a protein which has an amino acid sequence comprising substitution, deletion, insertion, addition or inversion of one or several amino acids other than the amino acid residue at position 139 in the amino acid sequence shown in SEQ ID NO: 2, or the amino acid sequence shown in SEQ ID NO: 2 in which the leucine residue at position 139 is changed to the amino acid residue other than the proline residue, said protein having a temperature-sensitive DTSR activity.

Specifically, the DNA includes a DNA which is:
(a) a DNA which comprises a nucleotide sequence of base numbers 359-1987 in a nucleotide sequence shown in SEQ ID NO: 1, or
(b) a DNA which hybridizes to the nucleotide sequence of base numbers 359-1987 in the nucleotide sequence shown in SEQ ID NO: 1 under stringent conditions and which encodes a protein having a temperature-sensitive DTSR activity.

Hereinafter, the above-mentioned DNA may be referred to as the "gene of the present invention" or "mutant type *dts*R gene". The protein encoded by the DNA may be referred to as the "mutant type DTSR protein".

The present invention also provides a coryneform bacterium having the gene of the present invention, having no wild type DTSR protein and having an ability to produce L-glutamic acid, and a method for producing L-glutamic acid, comprising the steps of cultivating the coryneform bacterium in a liquid medium to produce and accumulate L-glutamic acid in the medium, and collecting L-glutamic acid from the medium.

The present invention further provides the coryneform bacterium which further has an ability to produce L-lysine, and a method for producing L-lysine and L-glutamic acid, comprising the steps of cultivating the coryneform bacterium in a liquid medium to produce and accumulate L-lysine and L-glutamic acid in the medium, and collecting L-lysine and L-glutamic acid from the medium.

The DTSR activity used herein means an activity possessed by the DTSR protein, specifically, an activity contributing to a resistance to a surfactant in coryneform bacteria. For example, when the DTSR protein exists in cells of a surfactant-sensitive mutant of coryneform bacteria (a mutant of coryneform bacteria which deteriorates in growth in a medium containing a surfactant at a concentration at which the surfactant does not affect growth of a wild type coryneform bacterium), the DTSR protein has an activity of losing coryneform bacteria a sensitivity to a surfactant.

The temperature-sensitive DTSR activity means a property that the DTSR activity is shown at the optimum growth temperature (31.5°C) of coryneform bacteria but the DTSR activity is lowered at a temperature higher than a temperature of 33 to 37°C, preferably 34°C.

Coryneform bacteria as referred to herein are a group of microorganisms defined in Bergey's Manual of Determinative Bacteriology, 8th Ed., p. 599 (1974). The bacteria are aerobic, Gram-positive, non-acid-fast bacilli not having the ability to sporulate, and include bacteria which had been classified as bacteria belonging to the genus *Brevibacterium* but have now been unified into the genus *Corynebacterium* [see Int. J. Syst. Bacteriol., *41,* 255 (1981)] and also include bacteria of the genus *Brevibacterium* and *Microbacterium* which are closely related to the genus *Corynebacterium*.

### DETAILED DESCRIPTION OF THE INVENTION

The present invention will be described in detail hereinunder.

The gene of the present invention can be obtained by subjecting a recombinant DNA comprising a *dts*R gene originating from a wild type coryneform bacterium to mutagen treatment, introducing the treated DNA into a surfactant-sensitive mutant of coryneform bacteria, selecting a strain which grows at 31.5°C but does not grow at a higher temperature, for example, at 35°C in a medium containing a surfactant at a concentration at which a mutant into which the recombinant DNA is not introduced can not grow, and recovering the recombinant DNA from the obtained strain.

The *dts*R gene originating from the wild type coryneform bacterium can be obtained according to the method described in International Publication No. WO95/23224. A method for obtaining the wild type *dts*R gene by using a surfactant-sensitive mutant of coryneform bacteria and a method for obtaining the mutant type *dts*R gene by using the wild type *dts*R gene are described below.

Chromosomal DNA is recovered from the wild type strain of coryneform bacteria in accordance with a method of Saito et al. (H. Saito and K. Miura, *Biochem.* *Biophys. Acta, 72*, 619 (1963)). Recovered chromosomal DNA is digested with a restriction enzyme, and ligated with a vector functioning in coryneform bacteria to obtain various recombinant DNAs.

The vector functioning in coryneform bacteria is, for example, a plasmid which is autonomously replicable in coryneform bacteria. Specific examples of the vector are mentioned below.
(1) pAM 330 see Japanese Patent Application Laid-Open No. 58-67699 (1983)
(2) pHM 1519 see Japanese Patent Application Laid-Open No. 58-77895 (1983)
(3) pAJ 655 see Japanese Patent Application Laid-Open No. 58-192900 (1983)
(4) pAJ 611 see Japanese Patent Application Laid-Open No. 58-192900 (1983)
(5) pAJ 1844 see Japanese Patent Application Laid-Open No. 58-192900 (1983)
(6) pCG 1 see Japanese Patent Application Laid-Open No. 57-134500 (1982)
(7) pCG 2 see Japanese Patent Application Laid-Open No. 58-35197 (1983)
(8) pCG 4 see Japanese Patent Application Laid-Open No. 57-183799 (1982)
(9) pCG 11 see Japanese Patent Application Laid-Open No. 57-183799 (1982)

As the surfactant-sensitive mutant, it may be mentioned a mutant which grows worse than the corresponding wild type strain in a medium containing a surfactant at a concentration of from 0.1 to 1 mg/dl when the surfactant is polyoxyethylene sorbitan monopalmitate, for example. The growth of a wild type strain of a coryneform bacterium is not affected by the presence of the surfactant at a concentration of from 0.1 to 1 mg/dl in the medium. As one example of the surfactant-sensitive mutant of a coryneform bacterium, mentioned is *Corynebacterium glutamicum* AJ 11060. This mutant was deposited in the National Institute of Bioscience and Human-Technology, Agency of Industrial Science and Technology, Ministry of International Trade and Industry, under an accession number FERM P-3678.

In order to introduce the various recombinant DNAs into the surfactant-sensitive mutant strain belonging to the coryneform bacteria, a procedure may be carried out in accordance with conventional and reported transformation methods. For example, it is possible to use a method in which recipient cells are treated with calcium chloride to increase permeability of DNA as reported for *Escherichia coli* K-12 (Mandel, M. and Higa, A., *J. Mol. Biol., 53*, 159 (1970)), and a method in which competent cells are prepared from cells at a proliferating stage to introduce DNA thereinto as reported for *Bacillus subtilis* (Duncan, C. H., Wilson, G. A. and Yound, F. E., *Gene, 1*, 153 (1977)). Alternatively, it is also possible to apply a method in which DNA recipient cells are converted into a state of protoplasts or spheroplasts which easily incorporate recombinant DNA to introduce recombinant DNA into DNA recipients as known for *Bacillus subtilis*, actinomycetes, and yeast (Chang, S and Choen, S. N., *Molec. Gen. Genet., 168*, 111 (1979); Bibb, M. J., Ward, J. M. and Hopwood, O. A., *Nature, 274*, 398 (1978); Hinnen, A., Hicks, J. B. and Fink, G. R., *Proc. Natl. Acad. Sci. USA, 75*, 1929 (1978)). The transformation method used in the Examples of the present invention is an electric pulse method (Japanese Patent Application Laid-Open No. 2-207791 (1990)).

Then, obtained transformants are once spread on M-CM2G agar plates (containing glucose 5 g, polypeptone 10 g, yeast extract 10 g, NaCl 5 g, DL-methionine 0.2 g, agar 15 g, and chloramphenicol 4 mg in 1 l of pure water, pH 7.2) containing no surfactant to form tens of thousands of colonies. The colonies are replicated on M-CM2G plates containing 30 mg/L of a surfactant (Tween 40) to obtain those exhibiting good growth on the M-CM2G plates containing the surfactant. Thus strains with lost surfactant sensitivity can be obtained.

The same method as the method for preparing chromosomal DNA of wild type coryneform bacteria may be used to recover recombinant DNA from a transformed strain with lost surfactant sensitivity. The chromosomal DNA fragment from the wild type coryneform bacterium ligated to the vector is sequenced to confirm that the *dts*R gene is contained.

As described above, the *dts*R gene can be obtained.

The *dts*R gene can also be obtained amplifying a DNA fragment containing the *dts*R gene by PCR using oligonucleotides prepared based on the reported nucleotide sequence of the *dts*R gene as primers and coryneform bacterium chromosomal DNA as a template. Furthermore, the *dts*R gene can be obtained by screening a coryneform bacterium chromosomal library by hybridization using an oligonucleotide prepared based on the nucleotide sequence of the *dts*R gene as a probe.

A strain, *Escherichia coli* JM109/pDTR6 harboring pDTR6 containing the *dts*R gene (private number AJ12967) has been deposited on February 22, 1994 in National Institute of Bioscience and Human Technology of Agency of Industrial Science and Technology under an accession number of FERM P-14168, transferred to international deposition based on the Budapest Treaty on February 9, 1995, and awarded an accession number of FERM BP-4994.

The mutant type *dts*R gene can be obtained by subjecting DNA containing the wild type *dts*R gene to mutagenesis. Specifically, the DNA containing the wild type *dts*R gene is subjected to a treatment with a chemical reagent such as sodium hypochlorite and hydroxylamine (Shortle, D. and Nathans, D., *Proc. Natl. Acad. Sci. U.S.A., 75*, 270 (1978)). The treated recombinant DNA is introduced to a surfactant-sensitive mutant of coryneform bacteria to obtain transformants in the same manner as described above. From the transformants, a strain which grows at 31.5°C but does not grow at not less than a temperature of 33 to 37°C in a medium containing a surfactant at a concentration at which a non-transformed mutant cannot grow (e.g. medium containing 30 mg/mL polyoxyethylene sorbitan monopalmitate) is selected. From the selected strain, the recombinant DNA is recovered, and thus the mutant type *dts*R gene can be obtained.

As an example of the gene of the present invention obtained as described above, a nucleotide sequence of the *dts*R gene obtained by subjecting the *dts*R gene originating from *Brevibacterium lactofermentum* ATCC 13869 to mutation treatment is shown in SEQ ID NO: 1. Also, the mutant type DTSR protein encoded by the mutant type *dts*R gene is shown in SEQ ID NO: 2. The wild type DTSR protein originating from *Brevibacterium lactofermentum* ATCC 13869 has an amino acid sequence in which an amino acid residue at position 139 is a proline residue. The wild type *dts*R gene originating from *Brevibacterium lactofermentum* ATCC 13869 has a nucleotide sequence in which a base at position 774 is C.

Since an amino acid sequence of the mutant type DTSR protein having a temperature sensitivity and a nucleotide sequence of the mutant type *dts*R gene encoding the mutant type DTSR protein are clarified by the present invention, the mutant type *dts*R gene can be obtained also by means of a site-directed mutagenesis method (Kramer, W. and Frits, H. J., *Methods in Enzymology, 154*, 350 (1987)). Specifically, in the wild type *dts*R gene, a 139th codon for a proline residue in the amino acid sequence shown in SEQ ID NO: 1, may be replaced by a codon for an amino acid residue other than the proline residue, preferably a codon for a leucine residue. The amino acid residue other than the proline residue is not particularly limited provided that when the proline residue at position 139 is replaced with the amino acid residue, the mutant type DTSR protein has the temperature-sensitive DTSR activity. The amino acid residue other than the proline residue is preferably a leucine residue.

The gene of the present invention includes a DNA having a nucleotide sequence of base numbers 359-1987 in the nucleotide sequence shown in SEQ ID NO: 1 as well as a DNA encoding an amino acid sequence shown in SEQ ID NO: 2, and a DNA encoding an amino acid sequence in which a leucine residue at position 139 is changed to an amino acid residue other than a proline residue in the amino acid sequence shown in SEQ ID NO: 2. Even if a mutation resulting in substitution, deletion, insertion, addition or inversion of one or several amino acid residues at a position or positions other than the position 139 in the amino acid sequence shown in SEQ ID NO: 2 is added to the above-mentioned DNA, the DNA is included to the gene of the present invention provided that a protein encoded by the DNA has a temperature-sensitive DTSR activity.

The DNA encoding the protein having an amino acid sequence comprising substitution, deletion, insertion, addition or inversion of one or several amino acid residues at a position or positions other than the position 139 in the amino acid sequence shown in SEQ ID NO: 2 can be obtained by, for example, subjecting a coryneform bacterium having a *dts*R gene encoding a DTSR protein in which the amino acid residue at the position 139 is changed to an amino acid residue other than the proline residue, specifically, a *dts*R gene having the nucleotide sequence shown in SEQ ID NO: 1 to mutation treatment, and isolating a DNA which hybridizes to a DNA containing at least a part of the nucleotide sequence of base numbers 359-1987 in the nucleotide sequence shown in SEQ ID NO: 1 under stringent conditions from the obtained mutant. Also, the DNA can be obtained as a homologous or allelic variant.

Examples of the mutation treatment include ultraviolet irradiation and treatment with a mutating agent such as N-methyl-N'-nitro-N-nitrosoguanidine (NTG).

By the "stringent condition" referred to herein is meant a condition under which a specific hybrid is formed, and nonspecific hybrid is not formed. It is difficult to clearly express the condition with numerical values. However, the condition is exemplified by a condition under which, DNAs having high homology, for example, DNAs having homology of not less than 90% are hybridized with each other, and DNAs having homology lower than the above are not hybridized with each other, or a condition of a temperature of from a melting out temperature (Tm) of a completely-matched hybrid to (Tm - 30)°C, preferably from Tm to (Tm - 20)°C and a salt concentration corresponding to 1× SSC, preferably 0.1× SSC.

The DNA encoding the DTSR protein having an amino acid sequence comprising substitution, deletion, insertion, addition or inversion of one or several amino acid residues at a position or positions other than the position 139 can be obtained by modifying the nucleotide sequence to give substitution, deletion, insertion, addition or inversion of an amino acid residue or amino acid residues at a specified site by the site-specific mutagenesis.

A site to which mutation is introduced or in which mutation occurs can be determined by selecting a DNA encoding the DTSR protein which has the temperature-sensitive DTSR activity and whose amino acid sequence is mutated at a site other than the amino acid residue at position 139 from the DNAs obtained as described above. A site of the introduced mutation is not specifically restricted provided that no influence is substantially exerted on the temperature-sensitive DTSR activity. A number of the introduced mutation varies depending on a site or a kind of the mutated amino acid in a steric structure of a protein, and is not specifically restricted provided that no influence is substantially exerted on the temperature-sensitive DTSR activity. The number is usually 1 to 20, preferably 1 to 10.

The gene of the present invention can be suitably used for L-glutamic acid production. For example, an L-glutamic acid producing coryneform bacterium having the gene of the present invention and having no wild type DTSR protein has a temperature sensitivity to a surfactant. The coryneform bacterium can produce L-glutamic acid in a medium containing an excess amount of biotin without a surfactant when a cultivation temperature is raised higher than a temperature of 33-37°C after the cultivation at about 31.5°C to grow the coryneform bacterium.

Thus, according to the present invention, there are provided a coryneform bacterium having the gene of the present invention, having no wild type DTSR protein and having an ability to produce L-glutamic acid (the coryneform bacterium may be referred to as the "L-glutamic acid-producing bacterium of the present invention"), and a method for producing L-glutamic acid, comprising the steps of cultivating the coryneform bacterium in a liquid medium to produce and accumulate L-glutamic acid in the medium, and collecting L-glutamic acid from the medium.

The L-glutamic acid-producing bacterium of the present invention can be obtained by substituting a recombinant DNA comprising the gene of the present invention for the wild type *dts*R gene on chromosome of a coryneform bacterium having an ability to produce L-glutamic acid, by using an already established technique for homologous recombination (Experiments in Molecular Genetics, Cold Spring Harbor Laboratory press (1972); Matsuyama, S. and Mizushima, S., J. Bacteriol., 162, 1196(1985)) or the like. The coryneform bacterium having the ability to produce L-glutamic acid may be known ones (for example, see International Publication No. WO96/06180).

An ordinary nutrient medium containing a carbon source, a nitrogen source, inorganic salts, growth factors, etc. is used as the liquid medium for the cultivation of the aforementioned L-glutamic acid-producing bacterium of to the present invention. The L-glutamic acid-producing bacterium of the present invention has an ability to produce L-glutamic acid without allowing any biotin action-suppressing agent to be contained in a medium even in the case of cultivation in any liquid medium containing an excessive amount of biotin.

Carbohydrates such as glucose, fructose, sucrose, waste molasses, starch hydrolysate; alcohols such as ethanol and glycerol; and organic acids such as acetic acid may be used as the carbon source. Ammonium sulfate, ammonium nitrate, ammonium chloride, ammonium phosphate, ammonium acetate, ammonia, peptone, meat extract, yeast extract, corn steep liquor, etc. may be used as the nitrogen source. When an L-glutamic acid-producing bacterium having an auxotrophy is used, a required substance is added as a preparation or a natural material containing it.

Fermentation is performed for 2-7 days under an aerobic condition achieved by shaking cultivation, aerating and agitating cultivation or the like while maintaining pH of culture liquid at 5-9. pH is adjusted by using urea, calcium carbonate, ammonia gas, aqueous ammonia and the like. The cultivation temperature is 24-37°C. However, a better result is obtained by initiating cultivation at about 31.5°C, and raising the temperature to 33-40°C, preferably about 37°C at an intermediate stage of the cultivation. Namely, the bacterium is sufficiently proliferated in the vicinity of a temperature optimum for growth, and then the temperature is raised during cultivation. Thus production of L-glutamic acid is initiated without adding any biotin action-suppressing agent, and L-glutamic acid is produced and accumulated in a considerable amount in a culture liquid.

Collection of L-glutamic acid produced and accumulated in the culture liquid may be carried out in accordance with an ordinary method. For example, a method of ion exchange resin, a method of crystallization, etc. may be used. Specifically, L-glutamic acid is adsorbed and separated by using an anion exchange resin, or crystallized by neutralization.

The L-glutamic acid-producing bacterium of the present invention may further has an ability to produce L-lysine. L-Lysine and L-glutamic acid can be produced by cultivating such the coryneform bacterium (the coryneform bacterium may be referred to as the "L-lysine-producing bacterium of the present invention") in a liquid medium to produce and accumulate L-glutamic acid in the medium, and collecting L-glutamic acid from the medium.

The L-lysine-producing bacterium of the present invention can be obtained by substituting a recombinant DNA comprising the gene of the present invention for the wild type dtsR gene on chromosome of a coryneform bacterium having both of an ability to produce L-lysine and an ability to produce L-glutamic acid, by using homologous recombination or the like as described above. The coryneform bacterium having both of the ability to produce L-lysine and the ability to produce L-glutamic acid may be known ones (for example, see International Publication No. WO96/06180). Alternatively, the L-lysine-producing bacterium of the present invention can be obtained by giving an ability to produce L-lysine to the L-glutamic acid-producing bacterium by a method as described in Japanese Patent Publication No. 48-28078 (1983) or the like.

An ordinary nutrient medium containing a carbon source, a nitrogen source, inorganic salts, growth factors, etc., which is similar to that used for cultivation of the L-glutamic acid-producing bacterium of the present invention described above, is used as a liquid medium for cultivation of the L-lysine-producing bacterium of the present invention. The L-lysine-producing bacterium of the present invention has an ability to produce L-lysine and L-glutamic acid without allowing any biotin action-suppressing agent to be contained in a medium even in the case of cultivation in any liquid medium containing an excessive amount of biotin.

Fermentation is performed for 2-7 days under an aerobic condition achieved by shaking cultivation, agitating and aerating cultivation or the like while maintaining pH of culture liquid at 5-9. pH is adjusted by using urea, calcium carbonate, ammonia gas, aqueous ammonia and the like. The cultivation temperature is 24-37°C. However, a better result is obtained by initiating cultivation at about 31.5°C, and raising the temperature to 33-40°C, preferably about 37°C at an intermediate stage of the cultivation. Namely, L-lysine is mainly produced at about 31.5°C, but the rate of L-glutamic acid production is increased by raising the temperature during the cultivation. By utilizing this phenomenon, it is possible to control the ratio of L-lysine to L-glutamic acid in a culture liquid to be finally obtained as desired.

An ordinary method may be used for collecting L-lysine and L-glutamic acid produced and accumulated in the culture liquid. For example, a method of ion exchange resin, a method of crystallization, etc. may be used. When the method of ion exchange resin is used, L-lysine is firstly adsorbed and separated from the culture liquid by using a cation exchange resin, and then L-glutamic acid is adsorbed and separated by using an anion exchange resin, or crystallized by neutralization. When L-lysine and L-glutamic acid are used as a mixture, it is of course unnecessary to separate these amino acids with each other.

The L-glutamic acid productivity of the L-glutamic acid-producing bacterium and the L-lysine-producing bacterium of the present invention can be improved by enhancing genes of the glutamic acid biosynthesis system. The genes of the glutamic acid biosynthesis system having been enhanced in cells include, for example, phosphofructokinase of the glycolytic pathway (PFK, Japanese Patent Application Laid-open No. 63-102692 (1988)), phosphoenolpyruvate carboxylase of an anaplerotic pathway (PEPC, Japanese Patent Application Laid-open Nos. 60-87788 (1985) and 62-55089 (1987)), citrate synthase of the TCA cycle (CS, Japanese Patent Application Laid-open Nos. 62-201585 (1987) and 63-119688 (1988)), aconitate hydratase (ACO, Japanese Patent Application Laid-open No. 62-294086 (1987)), isocitrate dehydrogenase (ICDH, Japanese Patent Application Laid-open Nos. 62-166890 (1987) and 63-214189 (1983)), and glutamate dehydrogenase which catalyzes amination reaction (GDH, Japanese Patent Application Laid-open No. 61-268185 (1986)).

Also, the L-lysine productivity of the L-lysine-producing bacterium of the present invention can be improved by enhancing genes of the lysine biosynthesis system.

Known examples of the genes of the lysine biosynthesis system having been enhanced in cells include a gene coding for aspartokinase α-subunit protein or β-subunit protein in which concerted feedback inhibition by L-lysine and L-threonine is substantially desensitized (International Publication No. WO94/25605), a wild type phosphoenolpyruvate carboxylase gene originating from a coryneform bacterium (Japanese Patent Application Laid-open No. 60-87788 (1985)), a gene coding for wild type dihydrodipicolinate synthetase originating from a coryneform bacterium (Japanese Patent Publication No. 6-55149 (1994)), etc.

### Best Mode for Carrying Out the Invention

The present invention will be described in more detail with reference to the following examples.

### <1> Preparation of chromosomal DNA of Brevibacterium lactofermentum ATCC 13869 (a wild type strain of coryneform bacteria)

*Brevibacterium lactofermentum* ATCC 13869 was inoculated in 100 ml of a T-Y medium [containing 1% Bacto-trypton (by Difco), 0.5% Bacto-yeast extract (by Difco) and 0.5% NaCl; pH 7.2] and cultivated at 31.5°C for 8 hours to obtain a culture. This culture was subjected to centrifugation at 3,000 r.p.m. for 15 minutes to obtain 0.5 g of wet cells. From the wet cells, the chromosomal DNA was obtained according to Saito & Miura method (see *Biochem. Biophys. Acta. 72*, 619, (1963)). Next, 60 µg of the chromosomal DNA and 3 units of a restriction enzyme, *Sau*3AI were each mixed in 10 mM Tris-HCl buffer (containing 50 mM NaCl, 10 mM MgSO₄ and 1 mM dithiothreitol; pH 7.4), and the reaction was carried out at 37°C for 30 minutes. After the reaction, the reaction mixture was subjected to ordinary phenol extraction and ethanol precipitation to obtain 50 µg of chromosomal DNA fragments of *Brevibacterium lactofermentum* ATCC 13869 digested with *Sau*3AI.

### <2> Preparation of gene library of Brevibacterium lactofermentum ATCC 13869, using plasmid vector DNA

20 µg of a plasmid vector DNA (pSAC4) capable of autonomously replicating in both the cells of *Escherichia coli* and the cells of coryneform bacteria and 200 units of a restriction enzyme, *Bam*HI were mixed in 50 mM Tris-HCl buffer (containing 100 mM NaCl and 10 mM magnesium sulfate; pH 7.4) and reacted at 37°C for 2 hours to obtain a digested solution, and the solution was then subjected to ordinary phenol extraction and ethanol precipitation. After this, the DNA fragment was dephosphorylated with a bacterial alkaline phosphatase according to the method described in Molecular Cloning, 2nd Edition [J. Sambrook, E.F. Fritsch and T. Maniatis, Cold Spring Harbor Laboratory Press, p.1.56 (1989)] so as to prevent the re-binding of the plasmid vector-derived DNA fragment, and then the thus-dephosphorylated DNA fragment was subjected to ordinary phenol extraction and ethanol precipitation.

One µg of this pSAC4 digested with *Bam*HI, 1 µg of the chromosomal DNA fragments of *Brevibacterium lactofermentum* ATCC 13869 digested with *Sau*3AI, which was obtained in <1>, and 2 units of T4 DNA ligase (produced by Takara Shuzo Co., Ltd.) were added to 66 mM Tris-HCl buffer (pH 7.5) containing 66 mM magnesium chloride, 10 mM dithiothreitol and 10 mM ATP and reacted therein at 16°C for 16 hours to conduct the ligation of the DNA. Next, *Escherichia coli* DH5 were transformed with the DNA mixture by an ordinary method, and the resulting transformants were inoculated onto an L-agar medium containing 170 µg/ml of chloramphenicol to obtain about 20,000 colonies constituting a gene library.

### <3> Transformation of Brevibacterium lactofermentum AJ 11060 by gene library DNA

From these approximately 20,000 colonies mentioned above, the recombinant DNAs were recovered according to the above-mentioned Saito and Miura method.

The recombinant DNA mixture which was divided into 50 batches, was introduced into cells of the strain AJ 11060, a mutant being sensitive to surfactants, by ordinary transformation using electric pulse (see Japanese Patent Application Laid-Open No. 2-207791 (1990)). The resulting transformant cells were inoculated onto a glucose-added L-agar medium and the cultivation was performed by static incubation at 31.5°C, whereby about 20,000 colonies of transformants were formed on the medium. Next, these transformant colonies were replicated to the same plate medium but containing 30 mg/liter of a surfactant, and several strains that were resistant to the surfactant and grown on the plate medium were obtained therefrom.

Recombinant DNAs were each extracted from the obtained strains, and the strain AJ 11060 was transformed also with each of the recombinant DNAs. Consequently, strains resistant to the surfactant were obtained again. The recombinant DNA possessed by one of the strains was designated as pDTR6. Growth inhibition in a medium containing 3 g/l of a surfactant, of the pDTR6-introduced strain AJ 11060 was suppressed (see International Publication No. WO95/23224).

### <4> Preparation of mutant type dtsR gene

pDTR6 plasmid was subjected to a hydroxylamine treatment *in vitro* in accordance with a method described in a literature, Shortle, D. and Nathans, D., *Proc. Natl. Acad. Sci. U.S.A., 75*, 270 (1978), and it was introduced into AJ11060 by using the electric pulse method described above. About 20,000 strains of transformants were cultivated on an M-CM2G agar medium at 25°C for 30 hours to form colonies. The colonies on each plate were replicated on two plates of the medium containing 30 mg/l of a surfactant, followed by cultivation at 31.5°C and 35°C for 20 hours. After that, one strain was obtained which grew at 31.5°C but did not grow at 35°C. Plasmid was extracted from the strain in accordance with an ordinary method. Thus pDTR-117 was obtained.

### <5> Sequencing of mutant type dtsR gene

The plasmid pDTR-117 was introduced into *Escherichia coli* JM 109. The resulting *Escherichia coli* JM 109/pDTR-117 was cultivated in 20 ml of a medium containing 1% of tryptone, 0.5% of yeast extract and 0.5% of NaCl, at 37°C for 24 hours, and 20 ml of the resulting culture was inoculated in one liter of a medium having the same composition as above and cultivation was performed at 37°C for 3 hours. Then, 0.2 g of chloramphenicol was added thereto, and the cultivation was continued for additional 20 hours at the same temperature to obtain a culture. Next, the resulting culture was centrifuged at 3,000 r.p.m. for 10 minutes to obtain 2 g of wet cells. The obtained cells were suspended in 20 ml of 350 mM Tris-HCl buffer (pH 8.0) containing 25% of sucrose, and then 10 mg of lysozyme (produced by Sigma Co.), 8 ml of 0.25 M EDTA solution (pH 8.0) and 8 ml of 20% sodium dodecylsulfate solution were added thereto. Then, the resulting suspension was heated at 60°C for 30 minutes to obtain a lysate. 13 ml of 5 M NaCl solution was added to the lysate, and the lysate was then treated at 4°C for 16 hours. After the treatment, this was centrifuged at 15,000 r.p.m. for 30 minutes. The supernatant thus obtained was subjected to ordinary phenol extraction and ethanol precipitation to obtain a DNA precipitate.

The precipitate was dried under reduced pressure and then dissolved in 6 ml of 10 mM Tris-HCl buffer (pH 7.5) containing 1 mM EDTA, and 6 g of cesium chloride and 0.2 ml of ethidium bromide (19 mg/ml) were added thereto. Then, this was subjected to equilibrium density gradient centrifugation, using an ultracentrifugater, at 39,000 r.p.m. for 42 hours, by which the DNA was isolated. Next, ethidium bromide was removed from this, using n-butanol, and thereafter this was subjected to dialysis against 10 mM Tris-HCl (pH 7.5) containing 1 mM EDTA to obtain about 500 µg of a purified recombinant DNA, pDTR-117.

The nucleotide sequence of the cloned fragment of pDTR-117 was determined. The sequencing was effected according to the Sanger method, using a Taq DyeDeoxy Terminator Cycle Sequencing Kit (produced by Applied Biochemical Co.). The determined nucleotide sequence and an amino acid sequence deduced therefrom are shown in SEQ ID NO: 1 in the Sequence Listing. The amino acid sequence is also shown in SEQ ID NO: 2.

The sequencing showed that the cloned DNA fragment included the entire coding region of the *dts*R gene and that the base at position 774 which is C (cytosine) in the wild type *dts*R gene was replaced by T (thymine). By the substitution, in the encoded DTSR protein, the amino acid residue at position 139 which is a proline residue in the wild type DTSR protein is replaced by a leucine residue.

### <6> Construction of mutant type dtsR gene-introduced strains by gene substitution

Mutant type *dts*R gene-substituted strains were obtained in accordance with a homologous recombination method by using a temperature-sensitive plasmid as described in Japanese Patent Application Laid-open No. 5-7491 (1993). Specifically, pDTR6-117 described above was digested with *Xba*I and *Kpn*I, and the obtained fragment containing the *dts*R gene thereof was ligated with pHSG398 (produced by Takara Shuzo) having been digested with *Xba*I and *Kpn*I by using the method described above to obtain pHSGX-K-117.

Next, a plasmid pHSC4 (Japanese Patent Application Laid-open No. 5-7491 (1993)) having a mutant type replication origin with its temperature-sensitive autonomous replicability obtained from a plasmid autonomously replicable in coryneform bacteria was digested with restriction enzymes *Bam*HI and *Kpn*I to obtain a gene fragment containing the replication origin. The obtained DNA fragment was blunt-ended by using a DNA blunt end formation kit (produced by Takara Shuzo, Blunting kit), and then inserted into *Kpn*I recognition site of pHSGX-K-117 by using a *Kpn*I linker (produced by Takara Shuzo) to construct a plasmid pKTCX-K-117. *Escherichia coli* AJ12571 harboring pHSC4 has been deposited on October 11, 1990 in National Institute of Bioscience and Human Technology of Agency of Industrial Science and Technology under an accession number of FERM P-11763, transferred to international deposition based on the Budapest Treaty on August 26, 1991, and deposited under an accession number of FERM BP-3524.

The plasmid was introduced into *Brevibacterium lactofermentum* ATCC 13869 by using an electric pulse method, and the dtsR gene on chromosome was substituted into a mutant type in accordance with a method described in Japanese Patent Application Laid-open No. 5-7491 (1993). Specifically, *Brevibacterium lactofermentum* ATCC 13869/pKTCX-K-117 was cultivated with shaking in an M-CM2G liquid medium at 25°C for 6 hours, and then spread on an M-CM2G medium containing 5 µg/ml of chloramphenicol. Strains which formed colonies at 34 °C were obtained as plasmid-incorporated strains. Next, strains, which were sensitive to chloramphenicol at 34°C, were obtained from the plasmid-incorporated strains by using a replica method. No. 117 strain as a strain with lost surfactant resistance at 34°C was obtained from the sensitive strains. In the strain, the *dts*R gene on chromosome is substituted into a mutant type.

### <7> L-Glutamic acid productivity of No. 117 strain

The productivity of L-glutamic acid was evaluated for No. 117 strain obtained in the item <6>, as well as No. 11 strain, No. 77 strain and No. 21 strain described in International Publication No. WO96/06180 in the same manner as Example 2 described in International Publication No. WO96/06180. Specifically, the evaluation was conducted as follows.

*Brevibacterium lactofermentum* ATCC 13869 or each of the strains was inoculated to a seed culture medium having a composition shown in Table 1, and cultivated with shaking at 31.5°C for 24 hours to obtain a seed culture. A medium for full-scale cultivation having a composition shown in Table 6 was dispensed into each amount of 300 ml and poured into a jar fermenter made of glass having a volume of 500 ml, and sterilized by heating. After that, 40 ml of the seed culture was inoculated thereto. Cultivation was started by using an agitation speed of 800-1,300 rpm, an aeration amount of 1/2-1/1 vvm, and a cultivation temperature of 31.5°C. The culture liquid was maintained to have pH of 7.5 by using ammonia gas. The cultivation temperature was shifted to 37°C, 8 hours after the start of cultivation. A control for comparison was provided in which the cultivation temperature was not shifted, and cultivation was continued exactly at 31.5°C.

**Table 1**

| Component | Concentration | |
|---|---|---|
| | Seed culture | Full-scale culture |
| Glucose | 5 g/dl | 15 g/dl |
| KH₂PO₄ | 0.1 g/dl | 0.2 g/dl |
| MgSO₄·7H₂O | 0.04 g/dl | 0.15 g/dl |
| FeSO₄·7H₂O | 1 mg/dl | 1.5 mg/dl |
| MnSO₄·4H₂O | 1 mg/dl | 1.5 mg/dl |
| Soybean protein hydrolysate solution | 2 ml/dl | 5 ml/dl |
| Biotin | 50 µg/l | 200 µg/l |
| Thiamin hydrochloride | 200 µg/l | 300 µg/l |

In any experiment, the cultivation was finished at a point in time of 20-40 hours at which glucose was completely consumed. The amount of L-glutamic acid produced and accumulated in the culture liquid was measured.

As a result, the yield of L-glutamic acid of No. 117 strain was particularly improved as shown in Table 2.

**Table 2**

| Bacterial strain | L-Glutamic acid (g/dl) |
|---|---|
| ATCC 13869 | 0.5 |
| No. 11 | 7.5 |
| No. 77 | 6.9 |
| No. 21 | 8.3 |
| No. 117 | 9.0 |

### Industrial Applicability

According to the present invention, it is provided a mutant type *dts*R gene encoding a mutant type DTSR protein having a mutation of a temperature sensitivity to a surfactant. A coryneform bacterium having the mutant type DTSR protein and no wild type DTSR protein has a temperature sensitivity to a surfactant and can be used for L-glutamic acid production and the like.

## Claims

1. A DNA encoding:
(A) a protein which has an amino acid sequence shown in SEQ ID NO: 2, or an amino acid sequence shown in SEQ ID NO: 2 in which a leucine residue at position 139 is changed to an amino acid residue other than a proline residue, said protein having a temperature-sensitive DTSR activity, or
(B) a protein which has an amino acid sequence comprising substitution, deletion, insertion, addition or inversion of one or several amino acids other than the amino acid residue at the position 139 in the amino acid sequence shown in SEQ ID NO: 2, or the amino acid sequence shown in SEQ ID NO: 2 in which the leucine residue at the position 139 is changed to the amino acid residue other than the proline residue, said protein having a temperature-sensitive DTSR activity.

2. A DNA according to claim 1, which is:
(a) a DNA which comprises a nucleotide sequence of base numbers 359-1987 in a nucleotide sequence shown in SEQ ID NO: 1, or
(b) a DNA which hybridizes to the nucleotide sequence of base numbers 359-1987 in the nucleotide sequence shown in SEQ ID NO: 1 under strigent conditions and which encodes a protein having a temperature-sensitive DTSR activity where position 139 is not a proline.

3. A DNA according to claim 1, which encodes the protein which has the amino acid sequence shown in SEQ ID NO: 2.

4. A coryneform bacterium having the DNA as defined in any one of claims 1 to 3, having no wild type DTSR protein and having an ability to produce L-glutamic acid.

5. A coryneform bacterium according to claim 4, which further has an ability to produce L-lysine.

6. A method for producing L-glutamic acid, comprising the steps of:
cultivating the coryneform bacterium as defined in claim 4 in a liquid medium to produce and accumulate L-glutamic acid in the medium, and
collecting L-glutamic acid from the medium.

7. A method for producing L-lysine and L-glutamic acid, comprising the steps of:
cultivating the coryneform bacterium as defined in claim 5 in a liquid medium to produce and accumulate L-lysine and L-glutamic acid in the medium, and
collecting L-lysine and L-glutamic acid from the medium.

## Patentansprüche

1. DNA, codierend für:
(A) ein Protein, das eine Aminosäuresequenz hat, die in SEQ ID NO: 2 gezeigt ist, oder eine Aminosäuresequenz, die in SEQ ID NO: 2 gezeigt ist, in der ein Leucin-Rest der Position 139 in einen Aminosäurerest umgeändert ist, der von einem Prolin-Rest verschieden ist, wobei das Protein eine temperaturempfindliche DTSR-Aktivität aufweist, oder
(B) ein Protein, das eine Aminosäuresequenz hat, welche eine Substitution, Deletion, Insertion, Addition oder Inversion von einer oder mehreren Aminosäuren umfaßt, die nicht der Aminosäurerest sind, der an der Position 139 in Aminosäuresequenz, die in SEQ ID NO: 2 gezeigt ist, oder in der Aminosäuresequenz, die in SEQ ID NO: 2 gezeigt ist, in der der Leucin-Rest an der Position 139 in einen Aminosäurerest geändert worden ist, der nicht ein Prolin-Rest ist, wobei dieses Protein eine temperaturempfindliche DTSR-Aktivität aufweist.

2. DNA gemäß Anspruch 1, welche:
(a) eine DNA, die eine Nukleotidsequenz der Basennummern 359-1987 in der Nukleotidsequenz, die in SEQ ID NO: 1 gezeigt ist, umfaßt, oder
(b) eine DNA, die mit der Nukleotidsequenz der Basennummern 359-1987 in der Nukleotidsequenz, die in SEQ ID NO: 1 gezeigt ist, unter stringenten Bedingungen hybridisiert und die ein Protein mit einer temperaturempfindlichen DTSR-Aktivität codiert, wobei Position 139 nicht ein Prolin ist, ist.

3. DNA gemäß Anspruch 1, welche ein Protein codiert, das die Aminosäuresequenz hat, die in SEQ ID NO: 2 gezeigt ist.

4. Coryneformes Bakterium mit der DNA, die in einem der Ansprüche 1 bis 3 definiert ist, ohne Wildtyp DTSR-Protein und mit der Fähigkeit, L-Glutaminsäure zu produzieren.

5. Coryneformes Bakterium gemäß Anspruch 4, welches weiter die Fähigkeit besitzt, L-Lysin zu produzieren.

6. Verfahren zum Herstellen von L-Glutaminsäure, umfassend die Schritte:
Kultivieren des coryneformen Bakteriums, das in Anspruch 4 definiert wurde, in einem Flüssigmedium, um L-Glutaminsäure in dem Medium herzustellen und anzuhäufen, und
Einsammeln der L-Glutaminsäure aus dem Medium.

7. Verfahren zum Herstellen von L-Lysin und L-Glutaminsäure, umfassend die Schritte:
Kultivieren des coryneformen Bakteriums, wie in Anspruch 5 definiert, in einem Flüssigmedium, um L-Lysin und L-Glutaminsäure in dem Medium zu produzieren und anzuhäufen, und
Einsammeln von L-Lysin und L-Glutaminsäure aus dem Medium.

## Revendications

1. ADN codant pour :
(A) une protéine qui a une séquence d'acides aminés montrée dans SEQ ID NO : 2, ou une séquence d'acides aminés montrée dans SEQ ID NO: 2 dans laquelle un résidu leucine à la position 139 est changé en un résidu d'acide aminé autre qu'un résidu proline, ladite protéine ayant une activité DTSR thermosensible, ou
(B) une protéine qui a une séquence d'acides aminés comprenant une substitution, une délétion, une insertion, une addition ou une inversion de un ou plusieurs acides aminés autres que le résidu d'acide aminé à la position 139 dans la séquence d'acides aminés montrée dans SEQ ID NO : 2, ou la séquence d'acides aminés montrée dans SEQ ID NO : 2 dans laquelle le résidu leucine à la position 139 est changé en le résidu d'acide aminé autre que le résidu proline, ladite protéine ayant une activité DTSR thermosensible.

2. ADN selon la revendication 1, qui est :
(a) un ADN qui comprend une séquence de nucléotides des bases numéros 359 - 1987 dans une séquence de nucléotides montrée dans SEQ ID NO : 1, ou
(b) un ADN qui s'hybride à la séquence de nucléotides des bases numéros 359 - 1987 dans la séquence de nucléotides montrée dans SEQ ID NO : 1 dans des conditions stringentes et qui code pour une protéine ayant une activité DTSR thermosensible où la position 139 n'est pas une proline.

3. ADN selon la revendication 1, qui code pour une protéine qui a la séquence d'acides aminés montrée dans SEQ ID NO : 2.

4. Bactérie corynéforme ayant l'ADN tel que défini dans l'une quelconque des revendications 1 à 3, n'ayant aucune protéine DTSR de type sauvage et ayant une aptitude à produire de l'acide L-glutamique.

5. Bactérie corynéforme selon la revendication 4, qui a en outre une aptitude à produire de la L-lysine.

6. Procédé de production d'acide L-glutamique, comprenant les étapes :
de mise en culture de la bactérie corynéforme telle que définie dans la revendication 4 dans un milieu liquide pour produire et accumuler de l'acide L-glutamique dans le milieu, et
de récupération de l'acide L-glutamique du milieu.

7. Procédé de production de L-lysine et d'acide L-glutamique, comprenant les étapes :
de mise en culture de la bactérie corynéforme telle que définie dans la revendication 5 dans un milieu liquide pour produire et accumuler de la L-lysine et de l'acide L-glutamique dans le milieu, et
de récupération de la L-lysine et de l'acide glutamique du milieu.


### SEQUENCE LISTING

<110> AJINOMOTO Co., Inc.
<120> TEMPERATURE-SENSITIVE dtsR GENE
<150> JP 9-184176
   <151> 1997-07-09
<160> 2
<210> 1
   <211> 2047
   <212> DNA
   <213> Brevibacterium lactofermentum
<220>
   <221> CDS
   <222> 359..1987
<400> 1
   gatcttggaa ctcgacagtt ttcaccgtcc agtttggagc gcctgagctt gcaagctcca      60
   gcaagtcagc attagtggag cctgtcactt tttcgtaaat gacctggcca aagtcaccgt     120
   tttggagcaa tttttccttc aggagctcaa cgtttagcgg ctctctggat cgtgaaatgt     180
   caacgttcat ggaagccaat gtagtggggt cgcgtcgaaa agcgcgcttt aagggcgaca     240
   cgcccaaaaa gttttacctt taaaaactac ccgcacgcag cacgaacctg ttcagtgatg     300
   taaatcaccg cggaaatatt gtggacgtta cccccgccta ccgctacgat ttcaaaac       358
   atg acc att tcc tca cct ttg att gac gtc gcc aac ctt cca gac atc       406
   Met Thr Ile Ser Ser Pro Leu Ile Asp Val Ala Asn Leu Pro Asp Ile
    1               5                  10                  15
   aac acc act gcc ggc aag atc gcc gac ctt aag gct cgc cgc gcg gaa       454
   Asn Thr Thr Ala Gly Lys Ile Ala Asp Leu Lys Ala Arg Arg Ala Glu
               20                  25                  30
   gcc cat ttc ccc atg ggt gaa aag gca gta gag aag gtc cac gct gct       502
   Ala His Phe Pro Met Gly Glu Lys Ala Val Glu Lys Val His Ala Ala
           35                  40                  45
   gga cgc ctc act gcc cgt gag cgc ttg gat tac tta ctc gat gag ggc       550
   Gly Arg Leu Thr Ala Arg Glu Arg Leu Asp Tyr Leu Leu Asp Glu Gly
       50                  55                  60
   tcc ttc atc gag acc gat cag ctg gct cgc cac cgc acc acc gct ttc       598
   Ser Phe Ile Glu Thr Asp Gln Leu Ala Arg His Arg Thr Thr Ala Phe
   65                  70                  75                  80
   ggc ctg ggc gct aag cgt cct gca acc gac ggc atc gtg acc ggc tgg        646
   Gly Leu Gly Ala Lys Arg Pro Ala Thr Asp Gly Ile Val Thr Gly Trp
                       85                  90                  95
   ggc acc att gat gga cgc gaa gtc tgc atc ttc tcg cag gac ggc acc        694
   Gly Thr Ile Asp Gly Arg Glu Val Cys Ile Phe Ser Gln Asp Gly Thr
                   100                 105                 110
   gta ttc ggt ggc gcg ctt ggt gag gtg tac ggc gaa aag atg atc aag        742
   Val Phe Gly Gly Ala Leu Gly Glu Val Tyr Gly Glu Lys Met Ile Lys
               115                 120                 125
   atc atg gag ctg gca atc gac acc ggc cgc cta ttg atc ggt ctt tac        790
   Ile Met Glu Leu Ala Ile Asp Thr Gly Arg Leu Leu Ile Gly Leu Tyr
       130                 135                 140
   gaa ggc gct ggc gct cgc att cag gac ggc gct gtc tcc ctg gac ttc        838
   Glu Gly Ala Gly Ala Arg Ile Gln Asp Gly Ala Val Ser Leu Asp Phe
   145                 150                 155                 160
   att tcc cag acc ttc tac caa aac att cag gct tct ggc gtt atc cca        886
   Ile Ser Gln Thr Phe Tyr Gln Asn Ile Gln Ala Ser Gly Val Ile Pro
                   165                 170                 175
   cag atc tcc gtc atc atg ggc gca tgt gca ggt ggc aac gct tac ggc        934
   Gln Ile Ser Val Ile Met Gly Ala Cys Ala Gly Gly Asn Ala Tyr Gly
               180                 185                 190
   cca gcc ctg acc gac ttc gtg gtc atg gtg gac aag acc tcc aag atg        982
   Pro Ala Leu Thr Asp Phe Val Val Met Val Asp Lys Thr Ser Lys Met
           195                 200                 205
   ttc gtt acc ggc cca gac gtg atc aag acc gtc acc ggc gag gaa atc       1030
   Phe Val Thr Gly Pro Asp Val Ile Lys Thr Val Thr Gly Glu Glu Ile
       210                 215                 220
   acc cag gaa gag ctt ggc gga gca acc acc cac atg gtg acc gct ggc       1078
   Thr Gln Glu Glu Leu Gly Gly Ala Thr Thr His Met Val Thr Ala Gly
   225                 230                 235                 240
   aac tcc cac tac acc gct gcg acc gat gag gaa gca ctg gat tgg gta       1126
   Asn Ser His Tyr Thr Ala Ala Thr Asp Glu Glu Ala Leu Asp Trp Val
                   245                 250                 255
   cag gac ctg gtg tcc ttc ctc cca tcc aac aat cgc tct tac aca cca       1174
   Gln Asp Leu Val Ser Phe Leu Pro Ser Asn Asn Arg Ser Tyr Thr Pro
               260                 265                 270
   ctg gaa gac ttc gac gag gaa gaa ggc ggc gtt gaa gaa aac atc acc       1222
   Leu Glu Asp Phe Asp Glu Glu Glu Gly Gly Val Glu Glu Asn Ile Thr
           275                 280                 285
   gct gac gat ctg aag ctc gac gag atc atc cca gat tcc gcg acc gtt       1270
   Ala Asp Asp Leu Lys Leu Asp Glu Ile Ile Pro Asp Ser Ala Thr Val
       290                 295                 300
   cct tac gac gtc cgc gat gtc atc gaa tgc ctc acc gac gat ggc gaa       1318
   Pro Tyr Asp Val Arg Asp Val Ile Glu Cys Leu Thr Asp Asp Gly Glu
   305                 310                 315                 320
   tac ctg gaa atc cag gca gac cgc gca gaa aac gtt gtt att gca ttc      1366
   Tyr Leu Glu Ile Gln Ala Asp Arg Ala Glu Asn Val Val Ile Ala Phe
                   325                 330                 335
   ggc cgc atc gaa ggc cag tcc gtt gga ttt gtt gcc aac cag cca acc      1414
   Gly Arg Ile Glu Gly Gln Ser Val Gly Phe Val Ala Asn Gln Pro Thr
               340                 345                 350
   cag ttc gct ggc tgc ctg gac atc gac tcc tct gag aag gca gct cgc      1462
   Gln Phe Ala Gly Cys Leu Asp Ile Asp Ser Ser Glu Lys Ala Ala Arg
           355                 360                 365
   ttc gtc cgc acc tgc gac gcg ttt aac atc cca atc gtc atg ctt gtc      1510
   Phe Val Arg Thr Cys Asp Ala Phe Asn Ile Pro Ile Val Met Leu Val
       370                 375                 380
   gac gtc ccc ggc ttc ctt cca ggc gca ggc cag gag tat ggt ggc atc      1558
   Asp Val Pro Gly Phe Leu Pro Gly Ala Gly Gln Glu Tyr Gly Gly Ile
   385                 390                 395                 400
   ctg cgt cgt ggc gca aag ctg ctc tac gca tac ggc gaa gca acc gtt      1606
   Leu Arg Arg Gly Ala Lys Leu Leu Tyr Ala Tyr Gly Glu Ala Thr Val
                   405                 410                 415
   cca aag att acc gtc acc atg cgt aag gct tac ggc gga gcg tac tgc      1654
   Pro Lys Ile Thr Val Thr Met Arg Lys Ala Tyr Gly Gly Ala Tyr Cys
               420                 425                 430
   gtg atg ggt tcc aag ggc ttg ggc tct gac atc aac ctt gca tgg cca      1702
   Val Met Gly Ser Lys Gly Leu Gly Ser Asp Ile Asn Leu Ala Trp Pro
           435                 440                 445
   acc gca cag atc gcc gtc atg ggc gct gct ggc gca gtc gga ttc atc      1750
   Thr Ala Gln Ile Ala Val Met Gly Ala Ala Gly Ala Val Gly Phe Ile
       450                 455                 460
   tac cgc aag gag ctc atg gca gct gat gcc aag ggc ctc gat acc gta      1798
   Tyr Arg Lys Glu Leu Met Ala Ala Asp Ala Lys Gly Leu Asp Thr Val
   465                 470                 475                 480
   gct ctg gct aag tcc ttc gag cgc gag tac gaa gac cac atg ctc aac      1846
   Ala Leu Ala Lys Ser Phe Glu Arg Glu Tyr Glu Asp His Met Leu Asn
                   485                 490                 495
   ccg tac cac gct gca gaa cgt ggc ctg atc gac ggc gtg atc ctg cca      1894
   Pro Tyr His Ala Ala Glu Arg Gly Leu Ile Asp Gly Val Ile Leu Pro
               500                 505                 510
   agc gaa acc cgc gga cag att tcc cgc aac ctt cgc ctg ctc aag cac      1942
   Ser Glu Thr Arg Gly Gln Ile Ser Arg Asn Leu Arg Leu Leu Lys His
           515                 520                 525
   aag aac gtc act cgc cct gct cgc aag cac ggc aac atg cca ctg          1987
   Lys Asn Val Thr Arg Pro Ala Arg Lys His Gly Asn Met Pro Leu
   530 535 540
   taaatcggcg aatccataaa ggttcaaaag aattcaataa ggattcgata agggttcgat 2047
<210> 2
   <211> 543
   <212> PRT
   <213> Brevibacterium lactofermentum
<400> 2
   Met Thr Ile Ser Ser Pro Leu Ile Asp Val Ala Asn Leu Pro Asp Ile
    1               5                  10                  15
   Asn Thr Thr Ala Gly Lys Ile Ala Asp Leu Lys Ala Arg Arg Ala Glu
               20                  25                  30
   Ala His Phe Pro Met Gly Glu Lys Ala Val Glu Lys Val His Ala Ala
           35                  40                  45
   Gly Arg Leu Thr Ala Arg Glu Arg Leu Asp Tyr Leu Leu Asp Glu Gly
       50                  55                  60
   Ser Phe Ile Glu Thr Asp Gln Leu Ala Arg His Arg Thr Thr Ala Phe
   65                  70                  75                  80
   Gly Leu Gly Ala Lys Arg Pro Ala Thr Asp Gly Ile Val Thr Gly Trp
                   85                  90                  95
   Gly Thr Ile Asp Gly Arg Glu Val Cys Ile Phe Ser Gln Asp Gly Thr
               100                 105                 110
   Val Phe Gly Gly Ala Leu Gly Glu Val Tyr Gly Glu Lys Met Ile Lys
           115                 120                 125
   Ile Met Glu Leu Ala Ile Asp Thr Gly Arg Leu Leu Ile Gly Leu Tyr
       130                 135                 140
   Glu Gly Ala Gly Ala Arg Ile Gln Asp Gly Ala Val Ser Leu Asp Phe
   145                 150                 155                 160
   Ile Ser Gln Thr Phe Tyr Gln Asn Ile Gln Ala Ser Gly Val Ile Pro
                   165                 170                 175
   Gln Ile Ser Val Ile Met Gly Ala Cys Ala Gly Gly Asn Ala Tyr Gly
               180                 185                 190
   Pro Ala Leu Thr Asp Phe Val Val Met Val Asp Lys Thr Ser Lys Met
           195                 200                 205
   Phe Val Thr Gly Pro Asp Val Ile Lys Thr Val Thr Gly Glu Glu Ile
       210                 215                 220
   Thr Gln Glu Glu Leu Gly Gly Ala Thr Thr His Met Val Thr Ala Gly
   225                 230                 235                 240
   Asn Ser His Tyr Thr Ala Ala Thr Asp Glu Glu Ala Leu Asp Trp Val
                   245                 250                 255
   Gln Asp Leu Val Ser Phe Leu Pro Ser Asn Asn Arg Ser Tyr Thr Pro
               260                 265                 270
   Leu Glu Asp Phe Asp Glu Glu Glu Gly Gly Val Glu Glu Asn Ile Thr
   275 280 285
   Ala Asp Asp Leu Lys Leu Asp Glu Ile Ile Pro Asp Ser Ala Thr Val
   290 295 300
   Pro Tyr Asp Val Arg Asp Val Ile Glu Cys Leu Thr Asp Asp Gly Glu
   305 310 315 320
   Tyr Leu Glu Ile Gln Ala Asp Arg Ala Glu Asn Val Val Ile Ala Phe
   325 330 335
   Gly Arg Ile Glu Gly Gln Ser Val Gly Phe Val Ala Asn Gln Pro Thr
   340 345 350
   Gln Phe Ala Gly Cys Leu Asp Ile Asp Ser Ser Glu Lys Ala Ala Arg
   355 360 365
   Phe Val Arg Thr Cys Asp Ala Phe Asn Ile Pro Ile Val Met Leu Val
   370 375 380
   Asp Val Pro Gly Phe Leu Pro Gly Ala Gly Gln Glu Tyr Gly Gly Ile
   385 390 395 400
   Leu Arg Arg Gly Ala Lys Leu Leu Tyr Ala Tyr Gly Glu Ala Thr Val
   405 410 415
   Pro Lys Ile Thr Val Thr Met Arg Lys Ala Tyr Gly Gly Ala Tyr Cys
   420 425 430
   Val Met Gly Ser Lys Gly Leu Gly Ser Asp Ile Asn Leu Ala Trp Pro
   435 440 445
   Thr Ala Gln Ile Ala Val Met Gly Ala Ala Gly Ala Val Gly Phe Ile
   450 455 460
   Tyr Arg Lys Glu Leu Met Ala Ala Asp Ala Lys Gly Leu Asp Thr Val
   465 470 475 480
   Ala Leu Ala Lys Ser Phe Glu Arg Glu Tyr Glu Asp His Met Leu Asn
   485 490 495
   Pro Tyr His Ala Ala Glu Arg Gly Leu Ile Asp Gly Val Ile Leu Pro
   500 505 510
   Ser Glu Thr Arg Gly Gln Ile Ser Arg Asn Leu Arg Leu Leu Lys His
   515 520 525
   Lys Asn Val Thr Arg Pro Ala Arg Lys His Gly Asn Met Pro Leu
   530 535 540